Europäisches Patentamt

European Patent Office

Office européen des brevets

(11) Publication number: **0 411 619 A1**

# EUROPEAN PATENT APPLICATION

(21) Application number: **90114793.4**

(22) Date of filing: **01.08.90**

(51) Int. Cl.⁵: **A61K 31/415**, A61K 9/00, A61K 47/10

(30) Priority: **02.08.89 US 388786**

(43) Date of publication of application:
**06.02.91 Bulletin 91/06**

(84) Designated Contracting States:
**AT BE CH DE DK ES FR GB GR IT LI LU NL SE**

(71) Applicant: **WARNER-LAMBERT COMPANY**
**201 Tabor Road**
**Morris Plains New Jersey 07950(US)**

(72) Inventor: **Muhammad, Nouman**
**44 Center Grove Rd. Q23**
**Randolph, New Jersey 07869(US)**
Inventor: **Zour, Elena**
**71 Townsend Drive**
**Florham Park, New Jersey 07932(US)**
Inventor: **Mahjour, Majid**
**82 Koclas Drive**
**Netcong, New Jersey 07857(US)**
Inventor: **Lodhi, Shahid**
**441 Ratzer Road**
**Wayne, New Jersey 07470(US)**

(74) Representative: **Mansmann, Ivo**
**c/o Gödecke AG - Patentabteilung Postfach**
**569 Mooswaldallee 1-9**
**D-7800 Freiburg(DE)**

(54) Formulations of phenytoin sodium for intravenous administration.

(57) Formulations containing solutions of phenytoin sodium in water, in water and ethanol, or in water, ethanol and propylene glycol for intravenous administration.

EP 0 411 619 A1

## FORMULATIONS OF PHENYTOIN SODIUM FOR INTRAVENOUS ADMINISTRATION

### BACKGROUND

Phenytoin sodium (5,5-diphenyl-2,4-imidazolidinedione or sodium diphenylhydantoin) is a well-known anti-epileptic and anticonvulsant, Dilantin®. Its preparation is described in United States Patent 2,409,754, which is incorporated herein by reference.

Since phenytoin is poorly soluble in water, phenytoin sodium, which is more soluble, is employed in injectable preparations of the drug. In order to stabilize solutions of phenytoin sodium, various alcoholic solvent systems are used.

United States Patent 3,749,779 covers injectable solutions of sodium diphenylhydantoin in a stabilizing vehicle of from 70% to 100% propylene glycol with or without ethanol or glycerin, water, and monoethanolamine.

United States Patent 4,696,814 covers aqueous solutions of phenytoin sodium which can be stabilized against crystal formation by polyvinylpyrrolidone with or without alcohol solvent systems.

United States patent 4,642,316 covers phenytoin sodium complexes highly soluble in water. The complexes are formed by reacting, for example, choline hydroxide solution or ethylenediamine/sodium hydroxide solution with phenytoin sodium.

United States patent 3,856,964 covers sterilizable infusion concentrates containing 5,5-diphenylhydantoin sodium which can be diluted with standard aqueous infusion solutions.

One problem associated with intravenous injection of phenytoin sodium is the crystal formation which occurs upon dilution, local irritation and soft tissue necrosis at the site of administration. This effect is attributed to both high pH (12) and propylene glycol present in the formulation (Kilarski DJ, Buchanan MS, Behren LV: N Eng J Med 1984; 33:1186).

Thus there is a need for a preparation based on phenytoin which can be used for intravenous administration which eliminates the problems discussed above.

### SUMMARY OF THE INVENTION

The instant invention covers improved formulations/compositions of phenytoin sodium for intravenous administration which contain phenytoin sodium and a carrier. The carrier is water or water and ethanol or water, ethanol, and propylene glycol.

The composition contains about 40 mg/ml to about 60 mg/ml of phenytoin sodium. Preferably the composition contains about 50 mg/ml of phenytoin sodium.

The composition contains phenytoin sodium from about 1% to about 6% weight by volume, ethanol from about 0.0% to about 10% volume by volume, and propylene glycol from about 0.0% to about 40% volume by volume and water to make up to 100% as necessary.

Preferably the composition contains about 5% weight by volume phenytoin sodium, about 10% volume by volume ethanol, and about 10% volume by volume propylene glycol.

The pH of the composition is about 11.5 to about 12.5; preferably it is about 12.

### DESCRIPTION OF THE INVENTION

The instant invention covers intravenous compositions containing phenytoin sodium and a carrier.

Formulation can contain from 1 to 60 mg/ml phenytoin sodium. Preferably they contain from about 40 to about 60 mg/ml. The most preferred formulations contain about 50 mg/ml of phenytoin sodium. Formulations are prepared by adding a selected carrier and adjusting the pH of the solution to about 12 with any base suitable for an intravenous preparation. The preferred base is 1N NaOH.

Other solubilizing agents suitable for intravenous preparations are included in the instant invention.

Formulations, each containing 50 mg/ml of phenytoin sodium, were prepared (Table I) with water alone; water and 10% ethanol; and water, 10% ethanol, and 5% propylene glycol. Formulation A was prepared by adding the drug to water for injection and adjusting the pH of the solution to 12 with 1N NaOH. Formulation B was prepared by adding first the ethanol and then the drug to water for injection, while formulation C was prepared by adding, in order, ethanol, the propylene glycol and the drug.

The above solutions were then adjusted to pH 12 with 1N NaOH. All formulations were sparged with nitrogen throughout the manufacturing steps. Each solution was filtered through a 0.22 $\mu$m Durapore filter (Millipore) and was filled into 5cc clear glass ampules. The headspace of the samples was sparged with nitrogen before sealing the ampules.

Samples were placed on stability 1) at room temperature, 2) at 60°C, and 3) subjected to various stress conditions which included a) cycling between 4°C and 60°C for eight weeks and b) freezing to -15°C and thawing to room temperature.

The effect of terminal sterilization on the stability of the formulations was tested by autoclaving some samples for one hour at 121°C (Table II). The samples were monitored for their physical appearance, pH and chemical stability. Formulation A had observable crystals right away and Formulations B and C, after one week at room temperature. At these high temperatures extractables could leach out of the ampules and initiate formation of crystals.

In the instant invention the filtration technique was used to sterilize the product rather than terminal sterilization.

Visual inspection of samples subjected to the various stress conditions did not indicate any crystal formation. Particulate counts performed using a HIAC/Royco particulate counter showed all these formulations were within the USP limits for small volume parenterals (SVP) (Table III). Analysis of samples stored at 60°C for eight weeks did not show any significant decrease in the percent of the drug.

TABLE I

| Formulations Containing 50 mg/ml of Phenytoin Sodium | | | |
|---|---|---|---|
| Ingredient | Formulation | | |
| | A | B | C |
| Phenytoin sodium USP | 50mg | 50mg | 50mg |
| Ethanol USP | - | 10% | 10% |
| Propylene Glycol | - | - | 5% |
| NaOH 1N | q.s.pH:12 | q.s.pH:12 | q.s.pH:12 |
| Water for injection q.s. ad. | 1 ml | 1 ml | 1 ml |

TABLE II

| Analysis of Formulations Containing 50 mg/ml Phenytoin Sodium Autoclaved at 121°C for 1 Hour | | | |
|---|---|---|---|
| Formulation | pH | Appearance | % Remaining |
| A | 12.03 | Flat Crystals | 96.46 |
| B | 12.28 | No change* | 100.06 |
| C | 12.06 | No change* | 100.11 |

*Crystals were observed after 1 week at room temperature

TABLE III

| Particulate Counts of the Formulations Stored at Various Conditions | | | | |
|---|---|---|---|---|
| Time (weeks) | Particle size (μm) | Particulate counts per container of formulations stored at room temperature | | |
| | | A | B | C |
| 2 | 10 | 545 | 95 | 240 |
| | 25 | 45 | 5 | 15 |
| 5 | 10 | 345 | 1,105 | 1,835 |
| | 25 | 25 | 95 | 80 |
| 8 | 10 | 660 | 740 | 1,360 |
| | 25 | 60 | 20 | 45 |
| | | Particle counts per container of formulations stored at 60° C | | |
| 2 | 10 | 2,050 | 1,225 | 255 |
| | 25 | 275 | 165 | 25 |
| 5 | 10 | 1,090 | 650 | 355 |
| | 25 | 45 | 35 | 40 |
| 8 | 10 | 150 | 85 | 110 |
| | 25 | 20 | 10 | 10 |
| | | Particle counts per container of cycled formations | | |
| 2 | 10 | 120 | 300 | 555 |
| | 25 | 15 | 10 | 45 |
| 5 | 10 | 340 | 295 | 1,065 |
| | 25 | 15 | 40 | 115 |
| 8 | 10 | 2,550 | 1,455 | 1,250 |
| | 25 | 190 | 100 | 105 |
| USP specifications: | | | | |
| 10 μm <10,000 | | | | |
| 25 μm <1,000 | | | | |

As can be seen in Table III above, the particulate count was significantly below that permitted by USP values.

Table IV below shows that pH did not vary significantly after being subjected to various stress conditions.

4

TABLE IV

| The pH of Formulations on Stability at Various Conditions | | | |
|---|---|---|---|
| Time (weeks) | pH of formulations stored at room temperature | | |
| | A | B | C |
| 0 | 12.0 | 12.0 | 12.0 |
| 2 | 12.17 | 12.14 | 11.97 |
| 5 | 12.42 | 12.42 | 12.27 |
| 8 | 12.28 | 12.21 | 12.23 |
| | pH of formulations stored at 60°C | | |
| 0 | 12.0 | 12.0 | 12.0 |
| 2 | 12.44 | 12.43 | 12.02 |
| 5 | 12.22 | 12.22 | 12.00 |
| 8 | 11.98 | 12.04 | 11.83 |
| | pH of formulations cycled | | |
| 0 | 12.0 | 12.0 | 12.0 |
| 2 | 12.02 | 12.03 | 11.85 |
| 5 | 12.17 | 12.14 | 11.98 |
| 8 | 12.20 | 12.21 | 11.78 |

TABLE V

| Analysis of Formulations Containing 50 mg/ml Phenytoin Sodium Stored at 60°C (percent remaining) | | | |
|---|---|---|---|
| Time (weeks) | Formulation | | |
| | A | B | C |
| 0.0 | 100.0 | 100.0 | 100 |
| 2.0 | 101.43 | 103.69 | 105.05 |
| 5.0 | 99.43 | 105.5 | 106.07 |
| 8.0 | 97.93 | 102.08 | 105.16 |

Table V shows no degradation of phenytoin sodium occurred for any of the different formulations subjected to a stress condition of 60°C for up to 8 weeks.

EXAMPLES

Example 1

To 450 ml of water for injection purged with nitrogen, 25 g of phenytoin sodium was added, and the solution was stirred. The pH was adjusted to 12 with 1N NaOH, and the solution completed to volume by adding water for injection. The solution was sparged with nitrogen throughout the preparatory steps. Each solution was filtered through a 0.22 μm Durapore filter (Millipore). The solution was then placed in 5 ml clear glass ampules, the head space of the ampules purged with nitrogen, then sealed.

## Example 2

To 400 ml of water for injection purged with nitrogen, 50 ml absolute ethanol was added, and the solution was stirred. Then 25 g of phenytoin sodium was added and the solution stirred. The pH was then adjusted to 12 with 1N NaOH, and the solution completed to 500 ml with water for injection. The solution was then treated as in Example 1 above.

## Example 3

To 400 ml of water for injection purged with nitrogen, 50 ml absolute ethanol was added, and the solution stirred. Then 25 ml propylene glycol was added and the solution stirred. Then 25 g of phenytoin sodium was added with stirring. The pH was adjusted to 12 with 1N NaOH and the solution was completed to 500 ml with water for injection. The solution was then treated as in Example 1 above.

**Claims**

1. An intravenous composition containing phenytoin sodium and a carrier selected from the group consisting of:
   (a) water,
   (b) water and ethanol, or
   (c) water, ethanol, and propylene glycol.
2. A composition according to Claim 1 wherein phenytoin sodium concentration is from about 40 mg/ml to 60 mg/ml.
3. A composition according to Claim 2 wherein phenytoin sodium concentration is about 50 mg/ml.
4. A composition according to Claim 1 wherein phenytoin sodium is about 1% to about 6% weight by volume, ethanol about 0.0% to about 10% volume by volume, and propylene glycol is about 0.0% to about 40% volume by volume and water to make up to 100%.
5. A composition according to Claim 4 wherein phenytoin sodium is about 5% weight/volume, ethanol is about 10% volume by volume, and propylene glycol is about 10% volume by volume.
6. A composition according to Claim 1 wherein the pH is from about 11.5 to about 12.5.
7. A composition according to Claim 6 wherein the pH is about 12.
8. Process for the preparation of intra venous compositions comprising mixing the components according to claims 1 to 7.

European
Patent Office

EUROPEAN SEARCH
REPORT

Application Number

EP 90 11 4793

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | US-A-3 749 779 (G.H. SCHNELLER) <br> * Claims 1,7-9; column 4, lines 63-66 * <br> — — — | 1-4,6-8 | A 61 K 31/415 <br> A 61 K 9/00 <br> A 61 K 47/10 |
| D,Y | | 1-8 | |
| D,Y | EP-A-0 212 853 (WARNER-LAMBERT) <br> * Claims 1-2,5,8-9 * <br> — — — | 1-8 | |
| D,Y | FR-A-2 176 739 (DESITIN-WERK) <br> * Claims 1,6-9,11-12 * <br> — — — | 1-8 | |
| Y | EP-A-0 257 320 (BAYER) <br> * Claims 1,7-8; page 2, line 44; page 5, lines 9-12,21-24 * <br> — — — — — | 1-8 | |

TECHNICAL FIELDS
SEARCHED (Int. Cl.5)

A 61 K

The present search report has been drawn up for all claims

| Place of search | Date of completion of search | Examiner |
|---|---|---|
| The Hague | 06 November 90 | SCARPONI U. |